**Europäisches Patentamt**

**European Patent Office**

**Office européen des brevets**

(19)

(11) Veröffentlichungsnummer: **0 179 736 B1**

(12) **EUROPÄISCHE PATENTSCHRIFT**

(45) Veröffentlichungstag der Patentschrift: **31.07.91**

(51) Int. Cl.5: **A61F 2/34**

(21) Anmeldenummer: **85730147.7**

(22) Anmeldetag: **23.10.85**

(54) **Trägerelement für eine künstliche Hüftpfanne.**

(30) Priorität: **23.10.84 DE 8431422 U**

(43) Veröffentlichungstag der Anmeldung:
**30.04.86 Patentblatt 86/18**

(45) Bekanntmachung des Hinweises auf die
Patenterteilung:
**31.07.91 Patentblatt 91/31**

(84) Benannte Vertragsstaaten:
**CH DE FR GB IT LI SE**

(56) Entgegenhaltungen:
EP-A- 0 065 482     DE-A- 2 911 754
FR-A- 2 429 009     GB-A- 2 010 122
US-A- 3 608 096     US-A- 3 840 904

(73) Patentinhaber: **MECRON MEDIZINISCHE PRO-
DUKTE GMBH
Nunsdorfer Ring 23-27
W-1000 Berlin 48(DE)**

(72) Erfinder: **Anapliotis, Emmanuel
Tollensestrasse 16
W-1000 Berlin 37(DE)**

(74) Vertreter: **Christiansen, Henning, Dipl.-Ing.
Patentanwalt CHRISTIANSEN Pacelliallee
43/45
W-1000 Berlin 33(DE)**

**Beschreibung**

Die Erfindung betrifft ein Trägerelement (Schraubpfanne) der im Oberbegriff des Anspruchs 1 angegebenen Art.

Bei derartigen Trägerelementen, welche durch ihre in Form eines Gewindes angeordneten selbsttragenden Schneidflanken ohne Benutzung eines Gewindeschneiders in das entsprechend ausgefräste Acetabulum einschraubbar sind, sind bereits Sicherungen gegen ein unerwünschtes Verdrehen der implantierten Pfanne unter Belastung bekannt.

Bei einem aus der EP-A-0 065 482 bekannten Trägerelement wie auch bei einem solchen, das aus der FR-A-2 429 009 bekannt ist, sind die Tragflanken durch Schneidnuten unterbrochen, so daß durch die Stirnseiten der Tragflanken bereits eine gewisse Drehfixierung erfolgt.

Diese Fixierung ist jedoch in vielen Fällen nicht ausreichend, da die Tragflanken in Form eines selbstschneidenden Gewindes dazu bestimmt sind, in das Knochengewebe einzudringen - also von ihrer Funktion her keine Sperrwirkung ausüben sollen. Die Drehfixierung besteht also nur in dem Widerstand, welche dem Trägerelement beim selbstschneidenden Einschrauben vom Knochengewebe entgegengesetzt wird.

Der im Anspruch 1 angegebenen Erfindung liegt die Aufgabe zugrunde, eine zusätzliche Sicherung gegen ein unbeabsichtigtes Verdrehen der Pfanne unter Belastung vorzusehen.

Diese Aufgabe wird durch die im kennzeichnenden Teil des Anspruchs 1 angegebenen Maßnahmen gelöst.

Die Schwierigkeit bei der Lösung besteht insbesondere darin, daß die relativ großen Schneidkräfte beim Eindrehen der Schraubpfanne durch die Maßnahmen zur Verdrehsicherung nicht zusätzlich heraufgesetzt werden sollen. Andererseits sind die die Gewindesegmente bildenden mit Schneiden versehenen Tragflanken scharfkantig, so daß sie bei in Rotationsrichtung der Pfanne auftretenden Belastungen unter besonderen Umständen eine Pfannenlockerung dadurch hervorrufen können, daß sie Mikrobewegungen innerhalb der Gewindegänge ausführen. Die Fähigkeit der Flanke selbstschneidend zu wirken, steht dabei der Tendenz entgegen, daß die Pfanne durch Einschluß von Kallusmaterial in ihrer Rotationsbewegung gehemmt wird.

Die Erfindung beruht auf der Erkenntnis, daß eine langfristige Sicherung der Pfanne gegen Rotationsbewegungen im Zuge des "Einwachsens" am besten durch Bereiche zu erreichen ist, welche, wenn sie von Knochengewebe umschlossen sind, fest an diesen anhaften oder sperrend wirken, ohne daß die Pfanne in der Lage ist, sich infolge der in Rotationsrichtung wirkenden Kräfte von dieser Sperrwirkung zu befreien.

Insbesondere können in Hohlräume zwischen den genannten Oberflächenbereichen der Einschraubpfanne und der Innenoberfläche des Acetabulums Knochenspäne eingebracht werden, die das Anwachsen erleichtern. So werden beim selbstschneidenden Erzeugen des Gewindes im Acetabulum die durch die vorangeführten Kanten der Tragflanken ausgehobenen Späne sich in der eine vertiefte Aussparung bildenden Ausnehmung sammeln und beim weiteren Eintreiben der Pfanne mitgeführt bis diese ihre Endposition erreicht. In dieser Endposition wachsen die Knochenspäne an, wobei durch die Verbindung der somit in die Ausnehmung eingreifenden Knochenspäne mit dem Knochenmaterial durch jede Ausnehmung eine Sperre gegen unerwünschtes Verdrehen der Pfanne gegeben ist.

Die zusätzliche Sicherung der Pfanne im eingeschraubten Zustand ergibt sich aufgrund der Tatsache, daß die beim Einschrauben zunächst lose mitgeführten - oder nachträglich eingefügten - Knochenspäne der Spongiosa in der beim Einschrauben erreichten Endstellung der Pfanne zwischen den mit Gewindetragflanken versehenen Bereichen verbleiben und dort in der nunmehr erreichten Ruhestellung der Pfanne an das benachbarte Knochengewebe "anwachsen", indem sie in das sich neu bildende Knochengewebe am Pfannenrand einbezogen werden. Da das Pfannengewinde selbst relativ scharfkantig ist und somit durch bei Benutzung der Pfanne auftretenden Rotationsbewegungen kleiner Amplitude die Tendenz besteht, daß sich diese Segmente in das neu gebildete Gewebe einschneiden, ist es günstig, daß Elemente mit dem sich neu bildenden Knochengewebe in Verbindung kommen, welche eine oder mehrere Flächenteile aufweisen, deren Flächenvektor der tangentialen Bewegungsrichtung beim Ein- oder Ausschrauben entspricht.

Diese sich quer erstreckenden, die Schraubbewegung sperrenden Flächen bilden die Seitenflächen von wannenförmigen oder sonstigen Vertiefungen bzw. Durchbrechungen. Im Mikrobereich sind entsprechende Flächen bei in die Oberfläche eingelassenen bzw. darauf aufgetragenen Oberflächenrauhigkeiten vorhanden, wie sie als Madreporierung bekannt sind. Ein entsprechender Effekt läßt sich auch bevorzugt durch Plasmaspritzen von Titanmaterial auf die Oberfläche der Pfanne zwischen den Gewindesegmenten erreichen. Einen besonders günstigen Effekt hat auch die Kombination zweier oder mehrerer der vorgenannten Maßnahmen, so daß entsprechend den unterschiedlichen beim Einschrauben vorgefundenen Gegebenheiten jede der Maßnahmen in Abhängigkeit vom Einzelfall zur Auswirkung kommen kann.

Die genannten Maßnahmen kommen in Ihrer Anwendung insbesondere dann vorteilhaft zur Geltung, wenn der Arzt nach dem Einschrauben angefallene Knochenspäne in die vorgefundenen Hohlräume einfüllen und dort komprimieren kann. Dazu trägt insbesondere bei, daß die Tiefe der Schneidflanken sich vom größeren Pfannenquerschnitt zum kleineren hin verkleinert und infolge dessen im unteren Pfannenbereich nach dem Einschrauben ein Hohlraum zwischen dem Gewindegrund und der Innenoberfläche des Acetabulums besteht. Die Tendenz der Vergrößerung der Abmessungen dieses Hohlraumes zum unteren Bereich der Pfanne hin wird noch dadurch vergrößert, daß die Flanken entgengengesetzt zur Einschraubrichtung - auf Grund der selbstschneidenden Wirkung der Pfanne - weniger tief einschneiden.

Wenn die Vertiefungen als Durchbrüche ausgebildet sind, hat der Chirurg auch die Möglichkeit, vom Inneren der Pfanne her Knochenspäne einzupressen und mit dem Einschnappen des Pfanneneinsatzes das dort befindliche Material zu komprimieren.

Bei vorteilhaften Weiterbildungen der Erfindung erstrecken sich die Aussparungen quer zur Einschraubrichtung im wesentlichen über die gesamte Höhe der Anbringung der Tragflanken. Die Vertiefungen oder aufgerauhten Bezirke befinden sich dabei insbesondere in der Nähe von deren vorderen (Schneid)kanten. Dabei sind die wannenförmigen Vertiefungen insbesondere im Bereich ihrer Längsflanken relativ steil ausgebildet, wobei diese Steilbereiche an beiden Seitenwandungen entsprechend vorgesehen ist. Bei der in Einschraubrichtung nachgeführten Kante führt diese relativ steile Ausbildung dazu, daß die beim Einschneidvorgang losgelösten Späne sicher erfaßt werden und in die Ausnehmung gelangen, um dort festgehalten zu werden. Bei der gegenüberliegenden Längskante hingegen erzeugt eine relativ steile Ausbildung im Zusammenwirken mit den eingewachsenen Flanke einen besonders großen Widerstand gegen ein unbeabsichtigtes Ausschrauben der Pfannen. Diese Ausschraubbewegung ist die bevorzugt zu verhindernde unkontollierte Bewegung des Trägerelements.

Die günstigen Bemessungen der Pfanne mit den erfindungsgemäßen Eigenschaften ergeben sich wie folgt:
Der Kerndurchmesser der aus Titan gefertigten Pfanne beträgt an der Unterkante bevorzugt 38 bis 71 mm. Die Zähne springen an dieser Stelle - bezogen auf den Radius - um 2,8 mm vor, was der Gewindetiefe entspricht. Die Verringerung der Gewindetiefe vom unteren Pfannenrand nach obenhin wird dadurch erzeugt, daß ein Fräser, welcher eine Form aufweist, wie sie für die Erzeugung von genormten Knochenschraubengewinden erforderlich ist, entsprechend der sphärischen Form des Kerndurchmessers geführt - dabei aber nicht geneigt wird, sondern seine räumliche Richtung beibehält. Die einzelnen, die Gewindesegmente bildenden Tragflanken werden durch einen weiteren Fräsvorgang erzeugt, bei dem ein im wesentlichen kegelstumpfförmiger Fräser mit tangentialer Achsenrichtung - bezogen auf eine ringförmige Schnittfläche der Einschraubpfanne - auf einer vertikal dazu gerichteten der sphärischen Oberfläche der Pfanne folgenden Bahn verschoben wird. Der kegelstumpfförmige Teil des Fräsers ist bevorzugt ballig-konvex ausgebildet, so daß die abgeschrägten Rückflanken der Gewindesegmente konkav geformt sind und sich ein Bereich zwischen den Gewindesegmenten in vertikaler Richtung erstreckt, welcher im wesentlichen die Hälfte der Breite der benachbarten Gewindesegmente ausmacht und in dem von Segmenten befreiten Teil dem sphärischen Gewindegrund folgt.

Vorteilhafte Weiterbildungen der Erfindung sind in den Unteransprüchen gekennzeichnet bzw. werden nachstehend zusammen mit der Beschreibung von bevorzugten Ausführungen der Erfindung anhand der Zeichnung näher dargestellt. Es zeigen:
Figuren 1 bis 3 verschiedene Ausführungsformen der Erfindung in einander entsprechenden Darstellungen, jeweils in Seitenansicht, sowie
Figur 4 die Position der erfindungsgemäßen Pfanne im Acetabulum in der Ansicht von unten.

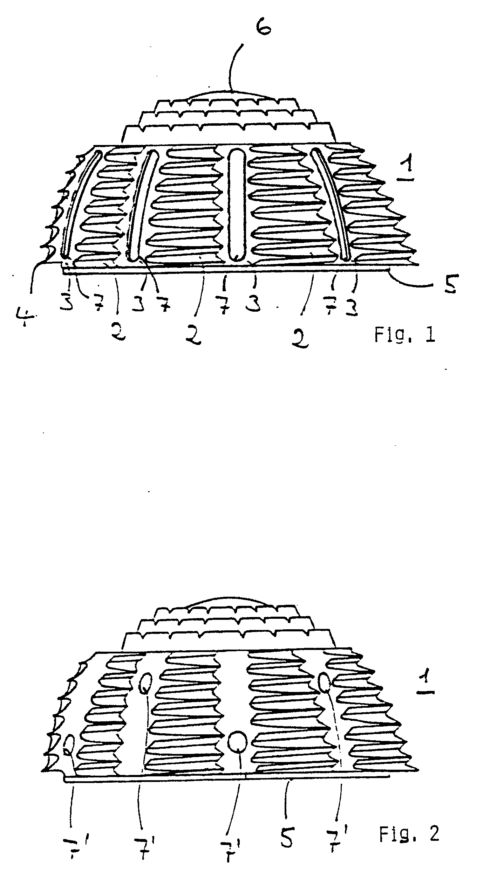

Bei dem in Figur 1 wiedergegebenen Trägerelement 1, welches ringförmig ausgebildet und dessen Außenfläche der Oberfläche einer Kugelzone entspricht, sind eine Anzahl von gewindeförmig angeordneten Tragflanken 2 vorgesehen, welche durch Schneidnuten 3 voneinander getrennt sind. Die Gewindesegmente bildenden Tragflanken sind an ihren beim Einschrauben vorangeführten Teilen scharfkantig ausgeführt und stehen hier am weitesten von der Kugeloberfläche ab. Sie bilden im Bereich der Schneidnuten scharfkantige Spitzen, welche beispielhaft mit 4 bezeichnet sind. In - bezogen auf die beim Einschrauben aufzuwendende Drehrichtung - weiter zurückliegenden Bereichen sind die Gewindesegmente zu ihren Außenkanten abgestumpft bis abgeflacht ausgebildet, was durch eine parallele Bearbeitung der übereinanderliegenden Gewindesegmente mittels Fräsen oder dgl. erfolgen kann. Das ringförmige Trägerelement besteht bevorzugt aus Titan, dessen Elastizitätseigenschaften bei geeigneter Querschnittsbemessung in etwa denen des umgebenden Knochenmaterials entsprechen.

Ein Pfanneneinsatz 5 ist an die inneren Querschnittsabmessungen des Trägerelementes 1 angepaßt und läßt sich in das bereits in den Knochen eingeschraubte Trägerelement von unten her einsetzen, wobei der obere kuppelförmige Bereich 6 des Einsatzes das Trägerelement überragt und sich im eingesetzten Zustand im Inneren der im Knochen angebrachten Aussparung abstützt.

Die Ausnehmungen 7 erstrecken sich - wie in der Figur ersichtlich - entlang der Vorderkanten der Tragflanken 4 im Bereich der Schneidnuten 3 senkrecht zur Einschraubrichtung des selbstschneidenden Trägerelements. Die Ausnehmungen bilden Vertiefungen, welche nicht vollständig den Querschnitt des metallenen Tragelements 1 durchqueren, so daß weiterhin eine hermetische Dichtung zwischen Pfanneninnerem und -äußerem gegeben ist und somit insbesondere die beim Einschraubvorgang anfallenden Späne nicht in das Pfanneninnere gelangen.

Die anfallenden Knochenspäne werden vielmehr in den wannenförmigen Vertiefungen 7 aufgefangen und dort gesammelt, wobei die bevorzugt steilflankigen Seitenflächen an den - in Einschraubrichtung gesehen - Hinterkanten ein sicheres Erfassen der Späne bewirken. Ist das Tragelement nach dem Einschrauben ruhiggestellt, wachsen die Knochenspäne an und verhindern das unbeabsichtigte Verdrehen der Pfanne - und insbesondere des unkontrollierte Ausschrauben -, wobei die steilen Flanken der in Einschraubrichtung vorangeführten Kanten im Zusammenwirken mit den angewachsenen Knochenspänen einen wirksame Hemmung bilden.

Die Vertiefungen weisen eine Breite von im wesentlichen 3 mm auf und eine Tiefe von 1 bis 1,5 mm, wobei die den Tragflanken benachbarten Kanten steilflankig ausgebildet und die Stirnseiten der Vertiefungen mit geringer Steigung in die Außenkontur der Pfanne übergehen.

In Figur 2 sind bei im übrigen der Figur 1 entsprechender Darstellung statt der Ausnehmungen Durchbrüche 7' dargestellt, welche zum Pfanneninneren hin durchgehen und abwechselnd im Bereich des unteren und des oberen Randes angeordnet sind. In diesen kreisrunden oder vertikal gestreckt ovalen Durchbrüchen sammeln sich die Knochenspäne beim Einschrauben in entsprechender Weise. Die Ränder der Durchbrüche 7' sind ebenfalls steil ausgebildet, so daß bei eingewachsenem Knochenmaterial ein sicherer Halt gegen Verdrehen gegeben ist. Bei den in den Innenraum durchgehenden Durchbrüchen besteht für den Chirurgen die Möglichkeit, vom inneren der Pfanne 1 her zusätzlich Knochenspäne in die Durchbrüche einzufügen, welche mit dem Vorgang des Einsetzens des Pfanneneinsatzes 5 komprimiert wird. Der bevorzugte Durchmesser der Durchbrüche beträgt 5 mm.

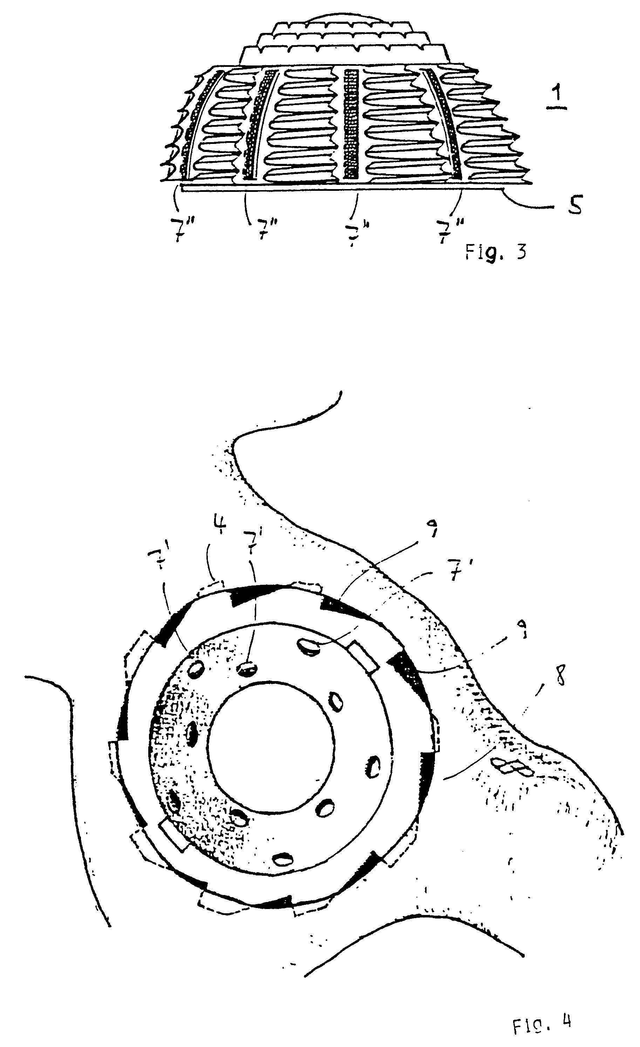

In Figur 3 sind die Bereiche zwischen den Tragflanken - bei im übrigen entsprechender Darstellung - mit aufgerauhten Zonen 7'' versehen, welche sich entsprechend den wannenförmigen Ausnehmungen in Figur 1 in vertikaler Richtung zwischen den mit Gewindesegmenten versehenen Bereichen erstrecken. Die Madreporierung kann entweder erhaben oder vertieft ausgebildet sein und entspricht in ihrer Gestaltung einer Anhäufung von Kugeln von 0,3 bis 0,7 mm Durchmesser in konvexer oder konkaver Ausformung. Die Bauchigkeit der Zone kann in einer anderen bevorzugten Ausführung auch durch Plasmaspritzen von Titanmaterial aufgebracht werden oder aber durch eine poröse Gestaltung der Oberflache durch Anätzen und dergleichen. Dabei liegt den Maßnahmen die Erkenntnis zugrunde, daß diese Bereiche beim Einschrauben der Pfanne in Kontakt mit der Oberfläche des Acetabulums zunächst nur eine geringe Reibung verursachen - wenn aber nach einiger Zeit das Knochenmaterial an die Pfannenoberfläche angeschlossen ist, haftet dieses in den betreffenden Bereichen fest an und bildet eine wirksame Drehsicherung.

Um die Wirksamkeit der Maßnahmen zur Verdrehsicherung gemäß Figuren 1 bis 3 noch zu verbessern, lassen sich diese Maßnahmen auch zu mehreren gemeinsam anwenden, wobei dazu entweder die genannten Maßnahmen in den Bereichen zwischen den Tragflanken nebeneinander vorgesehen sind - so insbesondere durchgehende Aussparungen neben aufgerauhten Bereichen - oder aber die Aufrauhungen sind neben und/oder in wannenförmigen Vertiefungen vorgesehen.

In Figur 4 ist die Pfanne 1 in der Ansicht von unten im in das Acetabulum 8 eingesetzten Zustand ohne Pfanneneinsatz wiedergegeben. Dabei ist insbesondere deutlich, daß - wegen der von der idealen Kreisform abweichenden Gestaltung des Acetabulums - Bezirke 9 vorhanden sind, in denen ein Abstand zwischen dem Kern der Pfanne 1 und der Innenoberfläche des Acetabulums besteht. In diesen Bereichen, welche von den erfindungsgemäßen Maßnahmen betroffen sind, lassen sich von unten her - wie ersichtlich - Knochenspäne nach dem Einschrauben der Pfanne einfüllen und komprimieren, so daß ein sicheres Anwachsen und die Verdrehsicherung zusätzlich unterstützt werden. Außerdem sind die nach innen durchgehende Durchbrüche 7' erkennbar, welche ebenfalls von innen her mit Knochenmaterial angefüllt werden können, bevor der Pfanneneinsatz 5 eingefügt wird.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch machen.

## Patentansprüche

1. In den Knochen einschraubbares sich in Einschraubrichtung verjüngendes Trägerelement für eine künstliche Hüftpfanne mit selbst-schneidenden, ein Gewinde bildenden Trag-flanken (2), wobei zwischen der beim selbstschneidenden Einschrauben vorangeführten Kante einer Tragflanke und der Hinterkante der vorangehenden Tragflanke ein Bereich des Trägerelements als Schneidnut freigelassen ist,

**dadurch gekennzeichnet,**

daß der als Schneidnut beigelassene Bereich mindestens eine Vertiefung (7,7') aufweist mit Flächenteilen, die im wesentlichen quer zur Bewegungsrichtung der Tragflanken beim Einschrauben gerichtet sind oder daß der als Schneidnut beigelassene Bereich mindestens eine solche Vertiefung (7,7') mit mindestens einer Oberflächenzone aufweist, die mit einer Aufrauhung, Porosität oder Madreporierung versehen ist.

2. Trägerelement nach Anspruch 1, mit durchge-henden Schneidnuten, **dadurch gekenn-zeichnet,** daß der Bereich (3) sich benachbart zu mehreren Tragflanken (2) entlang der Schneidnut erstreckt.

3. Trägerelement nach einem der vorangehenden Ansprüche, **dadurch gekennzeichnet,** daß der Bereich (3) im wesentlichen die halbe Breite der benachbarten Tragflanken (2) aufweist.

4. Trägerelement nach einem der vorangehenden Ansprüche, **dadurch gekennzeichnet,** daß die Vertiefung (7) wannenförmigen ausgebildet ist.

5. Trägerelement nach Anspruch 4, **dadurch gekennzeichnet,** daß die beiden Längskanten der wannenförmigen Vertiefung (7) steilflankig ausgebildet sind.

6. Trägerelement nach einem der vorangehenden Ansprüche, **dadurch gekennzeichnet,** daß die Vertiefung (7') einen Durchbruch zum Pfanneninneren hin von insbesondere rundem Querschnitt bildet.

7. Trägerelement nach Anspruch 6, **dadurch ge-kennzeichnet,** daß Durchbrüche als kreisrun-de Löcher abwechselnd in der Nähe des oberen und des unteren Randes vorgesehen sind.

8. Trägerelement nach einem der vorangehenden Ansprüche, **dadurch gekennzeichnet,** daß Aufrauhungen (7'') durch Plasmaspritzen und/oder Anätzen erzeugt sind.

9. Trägerelement nach einem der vorangehenden Ansprüche, **dadurch gekennzeichnet,** daß die äußere Oberfläche mindestens im Gewindegrund sphärisch ausgebildet ist.

10. Trägerelement nach einem der vorangehenden Ansprüche, **dadurch gekennzeichnet,** daß die Tiefe der Gewindesegmente (Gewindetiefe) in Einschraubrichtung zunimmt.

11. Trägerelement nach einem der vorangehenden Ansprüche, **dadurch gekennzeichnet,** daß die Tragflanken (2) als Gewindesegmente nach Art eines Knochenschraubengewindes ausgebildet sind.

**Claims**

1. Support element for an artificial acetabulum, which support element can be screwed into the bone, tapers in the direction of screwing-in and has self-tapping support flanks (2) which form a thread, an area of the support element being left free as a cutting groove between the edge of one support flank advanced during the self-tapping screwing-in and the rear edge of the preceding support flank, characterised in that the area left free as a cutting groove has at least one recess (7, 7') with surface parts which are directed essentially transverse to the direction of movement of the support flanks during the screwing-in, or in that the area left free as a cutting groove has at least one such recess (7, 7') with at least one surface zone which is provided with roughening, porosity or a madrepore finish.

2. Support element according to Claim 1, with continuous cutting grooves, characterised in that the area (3) extends adjacent to several support flanks (2) along the cutting groove.

3. Support element according to one of the preceding claims, characterised in that the area (3) has essentially half the width of the adjacent support flanks (2).

4. Support element according to one of the preceding claims, characterised in that the recess (7) is designed in a trough shape.

5. Support element according to Claim 4, characterised in that the two longitudinal edges of the trough-shaped recess (7) are designed with steep flanks.

6. Support element according to one of the preceding claims, characterised in that the recess (7') forms a passage of in particular round cross-section towards the inside of the acetabulum.

7. Support element according to Claim 6, characterised in that passages are provided as circular holes alternately in the vicinity of the upper and of the lower edge.

8. Support element according to one of the preceding claims, characterised in that roughened areas (7'') are produced by plasma spraying and/or etching.

9. Support element according to one of the preceding claims, characterised in that the outer surface is designed spherically at least in the thread base.

10. Support element according to one of the preceding claims, characterised in that the depth of the thread segments (thread depth) increases in the direction of screwing-in.

11. Support element according to one of the preceding claims, characterised in that the support flanks (2) are designed as thread segments in the manner of a bone-screw thread.

## Revendications

1. Elément porteur pour cupule de prothèse de la hanche s'effilant dans la direction de vissage, vissable dans les os, avec flancs supports autocoupants formant un filetage (2), où entre le bord d'un flanc support introduit dans le vissage autocoupant et le bord postérieur du flanc support précédent une zone de l'élément porteur est laissée libre comme rainure de coupure, caractérisé en ce que la zone laissée libre comme rainure de coupure présente au moins une cavité (7,7') avec des éléments de surface qui sont pour l'essentiel orientés perpendiculairement à la direction de mouvement des flancs supports lors du vissage, ou en ce que la zone laissée libre comme rainure de coupure présente au moins une cavité (7,7') de ce type avec au moins une zone superficielle pourvue d'une rugosité, porosité ou madréporation.

2. Elément porteur selon la revendication 1, avec rainures de coupure continues, caractérisé en ce que la zone (3) s'étend au voisinage de plusieurs flancs supports (2) le long de la rainure de coupure.

3. Elément porteur selon l'une des revendications précédentes, caractérisé en ce que la zone (3) présente pour l'essentiel la moitié de la largeur des flancs supports (2) voisins.

4. Elément porteur selon l'une des revendications précédentes, caractérisé en ce que la cavité (7) a une forme de cuvette.

5. Elément porteur selon la revendication 4, caractérisé en ce que les deux bords longitudinaux de la cavité en forme de cuvette (7) ont des flancs à forte pente.

6. Elément porteur selon l'une des revendications précédentes, caractérisé en ce que la cavité (7') forme un percement à l'intérieur de la cupule de section en particulier ronde.

7. Elément porteur selon la revendication 6, caractérisé en ce que des percements sont prévus en forme de trous circulaires alternativement au voisinage du bord supérieur et du bord inférieur.

8. Elément porteur selon l'une des revendications précédentes, caractérisé en ce que des rugosités (7'') sont produites par des pulvérisations de plasma et/ou décapages.

9. Elément porteur selon l'une des revendications précédentes, caractérisé en ce que sa surface externe est de forme sphérique au moins au fond du filetage.

10. Elément support selon l'une des revendications précédentes, caractérisé en ce que la profondeur des segments de filetage (profondeur de filetage) croît dans la direction de vissage.

11. Elément porteur selon l'une des revendications précédentes, caractérisé en ce que les flancs support (2) sont formés en segments de filetage à la manière d'un filetage pour vissage d'os.

6

1

3 7 3 7 7 3 7 3

4

2 2 2

Fig. 1

1

7' 7' 7' 5 7' Fig. 2

Fig. 3

FIG. 4